# EUROPEAN PATENT SPECIFICATION

(11) **EP 2 467 068 B1**
(45) Date of publication and mention of the grant of the patent: **21.10.2015**
(21) Application number: 10749548.3
(22) Date of filing: 20.08.2010
(51) Int. Cl.: A61B 17/12, A61B 17/00

(54) **EMBOLIC COIL INTRODUCER CATHETER LOCKING MECHANISMS**
KATHETERBLOCKMECHANISMEN FÜR EMBOLIESPIRALENEINFÜHRER
MÉCANISMES DE VERROUILLAGE D UN CATHÉTER D INTRODUCTION DE BOBINE EMBOLIQUE

(30) Priority: 20.08.2009 US 235397 P; 10.08.2010 US 853965
(43) Date of publication of application: 27.06.2012
(73) Proprietor: Boston Scientific Scimed, Inc., Maple Grove, MN 55311 (US)
(72) Inventor: GUO, Lantao, San Ramon California 94582 (US); DAO, Jimmy, San Jose California 95133 (US)
(74) Representative: Vossius & Partner Patentanwälte Rechtsanwälte mbB
(86) International application number: PCT/US2010/046221
(87) International publication number: WO 2011/022691

(56) References cited:
- EP-A1- 1 728 477
- WO-A1-2008/085606
- WO-A2-2008/064205
- WO-A2-2008/064206

## Description

### FIELD OF THE INVENTION

The invention pertains to embolic coil introducer catheter locking mechanisms, components and methods of use thereof.

### BACKGROUND

Therapeutic vascular occlusions arc used to prevent or treat pathological conditions, such as aneurysms, in situ. Embolic coils are often used to provide such therapeutic vascular occlusions in a variety of situations. Some embolic coils are introduced to the region of interest in a generally straightened condition inside a delivery catheter. The delivery sheath may be advanced to the region of interest, and the embolic coil may then be pushed out the distal end of the delivery catheter using a pusher wire, which may be removably attached to the embolic coil. The embolic coil typically assumes its final, coiled configuration as it is pushed out the distal end of the delivery catheter.

WO 2008/085606 discloses a device for delivering an occlusive element that includes an elongate pusher member having a lumen extending between distal and proximal ends. An elongate releasing member is slideably disposed within the lumen of the elongate pusher member. A retaining member is disposed on the distal end of the elongate pusher member and includes a finger portion having an aperture sized to receive the distal end of the elongate releasing member. An occlusive element such as a coil is provided and includes a securing member positioned at a proximal end thereof. The securing member is releaseably secured to the finger portion of the retaining member. The securing member is secured to the finger portion when the elongate releasing member is disposed in the aperture of the finger portion and unsecured when the elongate releasing member is retracted proximally from the aperture of the finger portion.

WO 2008/064206 discloses a device for delivering an occlusive element that includes an elongate pusher member having a lumen. A locking member is disposed within the lumen of the elongate pusher member. A moveable elongate releasing member is disposed within the lumen of the elongate pusher member. A filament is secured to the distal end of the elongate releasing member. The occlusive member is locked to the elongate releasing member when the filament passes through a securing member on the occlusive member and is pinched or wedged between the locking member and the elongate releasing member. The occlusive element is in an unlocked state when the elongate releasing member is retracted proximally relative to the elongate pusher member.

### SUMMARY

Embodiments of the invention pertain to embolic coil introducer catheter locking mechanisms, as well as related components, systems and methods. In many instances, it may be desirable to lock the relative positions of the catheter to the introducer wire during certain steps of a procedure, and then allow the introducer wire to move relative to the catheter during other steps of a procedure. Embodiments of the invention pertain generally to locking mechanisms of simple construction and easy use. In many cases, no additional steps are needed to use the locking systems of certain embodiments.

In one aspect, embolic coil introducer systems are provided wherein a catheter includes a locking mechanism that provides a friction fit between the catheter and the introducer wire to hold the wire secure relative to the catheter in a first position when no force is applied to the wire and wherein the friction fit may be overcome by the application of a force to the wire to allow the wire to move relative to the catheter.

In a second aspect, the locking mechanism may be configured to automatically hold the wire secure relative to the catheter in a second position after force has been applied to the wire to move the wire relative to the catheter, after the force has then been removed, wherein the locking mechanism may be configured such that the force needed to overcome the friction fit is greater than 0.22 N and less than 0.58 N (greater than 0.05 lbs and less than 0.13 lbs).

In a third aspect, the locking mechanism may be distal to the proximal end and in the proximal third of the catheter.

In a fourth aspect, the locking mechanism may be a reduced profile section of the catheter. For example, the locking mechanism may be a section of the catheter having a second inner diameter that is less than the first inner diameter. The catheter may include a stepwise transition between the first inner diameter and the locking mechanism or may include a tapering section between the first inner diameter and the locking mechanism.

In a fifth aspect, the locking mechanism may define a lumen for receiving the wire wherein the cross-sectional shape of the lumen is noncircular, such as a cross-sectional shape that is generally oval. The lumen may be configured to contact the wire along two, three, four, five or more noncontiguous inner surfaces of the locking mechanism, and the noncontiguous inner surfaces of the locking mechanism may have a greater dimension along the direction of the longitudinal axis than in a circumferential direction and may be generally uniformly disposed in a circumferential direction around the locking mechanism.

In a sixth aspect, the locking mechanism may include a first section that provides a friction fit with the wire and a second section that provides a friction fit with the wire separated longitudinally from each other by an intermediate section that does not provide a friction fit with the wire or is otherwise fixed to the wire.

In a seventh aspect, the locking mechanism may be a unitary component of the catheter. For example, the locking mechanism may be formed by crimping the catheter or may be formed from a section of the catheter by applying heat and pressure to that section of the catheter.

In an eighth aspect, the locking mechanism may have only a single configuration such that the locking mechanism components are not movable between a first and a second configuration because there is only a single configuration.

In a further aspect, the system may include a removable element disposed at the proximal end of the catheter to fix the relative longitudinal position of the catheter with the wire, such as a piece of heat shrink tubing, and the removable element may include either a slit and a perforation.

In a further aspect, the locking mechanism may be an insert fixed to the catheter.

In a further aspect, embolic coil introducer systems are provided wherein the catheter may include a non-removable locking mechanism that has essentially the same size and shape in a first position where the catheter is locked to the wire as in a second position where the wire is sliding relative to the catheter. There may be an adhesion bond between the locking mechanism and the wire in the first position, or there may be a friction fit between the locking mechanism and the wire in the first position. There may further be a friction fit between the locking mechanism and the wire in a third position where the wire is static relative to the catheter.

In a further aspect, embolic coil introducer systems are provided wherein the catheter may include a non-removable locking mechanism such that contact between the catheter and the wire fixes the wire in place when less than 0.044 N, 0.027 N, 0.013 N (0.010 lbs., 0.006 lbs. or 0.003 lbs. of force) are applied to the wire.

The above summary is not intended to describe each disclosed embodiment or every implementation of the invention.

### BRIEF DESCRIPTION OF THE DRAWINGS

FIG. 1 is a partial cross-sectional schematic view depicting an embolic coil introducer system;
FIG. 2 is a partial cross-sectional schematic view depicting a proximal portion of an embolic coil introducer system;
FIG. 3A is a cross-sectional schematic view of a locking mechanism for use in an embolic coil introducer system;
FIG. 3B is a cross-sectional schematic view of a locking mechanism for use in an embolic coil introducer system;
FIG. 4 is a partial cross-sectional schematic view depicting a proximal portion of an embolic coil introducer system; and
FIG. 5 is a partial cross-sectional schematic view to taking a proximal portion of an embolic coil introducer system.

### DESCRIPTION OF SELECTED EMBODIMENTS

All numeric values are herein assumed to be modified by the term "about", whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

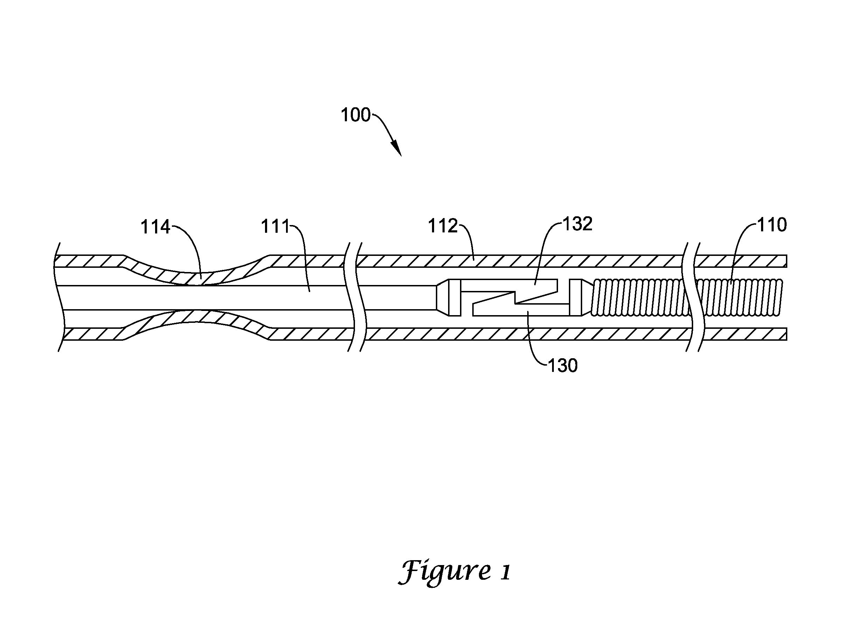

FIG. 1 depicts an example embolic coil delivery system 100 used to deliver a detachable embolic coil 110 to fill and occlude an aneurysmal sac. Embolic coil delivery system 100 includes an embolic coil 110 disposed within a catheter 112. Embolic coil 110 is preferably detachably engaged to delivery wire 111, which extends proximally out of catheter 112. Embolic coil 110 may be disposed within a carrier fluid such as a saline solution, a contrast agent, or a heparin solution, while embolic coil 110 is within catheter 112. An end fitting 130 of embolic coil 110 is detachably engaged within an end fitting 132 of delivery wire 111.

Embolic coil delivery systems and detachable embolic coils are described, for example, in U.S. Patent Pub. No. 2008/0221554 to O'Connor et al., filed on Feb. 12, 2008; U.S. Pat. No. 5,304,195 to Twyford, Jr. et al.; U.S. Pat. No. 5,895,385 to Guglielmi et al.; and U.S. Patent Pub. No. 2007/0141099 to Buiser et al., filed on Dec. 19, 2005.

Catheter 112 includes toward its proximal end a locking mechanism 114. In this embodiment, locking mechanism 114 is a narrowed section of catheter 112. Catheter 112 gradually reduces in size to the locking mechanism. Locking mechanism 114 is sized to contact wire 111 and hold wire 111 in place. Such contact may be, for example, a friction fit which applies force to the wire to hold it in place. The locking mechanism may be sized so that a particular amount of force applied to the wire may overcome the effect of the contact to allow the wire slide relative to the catheter. The amount of force needed to overcome the locking mechanism may be in the range of about 0.004 - 0.133 N, about 0.009 - 0.089 N, about 0.013 - 0.053 N, about 0.013 - 0.04 N, about 0.017 - 0.031 N (about 0.001-0.030 lbs, about 0.002-0.020 lbs, about 0.003-0.012 lbs, about 0.003-0.009 lbs, or about 0.004-0.007 lbs, respectively). The amount of force needed to overcome the locking the mechanism is preferably sufficient to prevent the inadvertent release of the wire from the catheter yet not so great as to prevent an operator from manually overcoming the locking mechanism.

As the locking mechanism 114 needs no direct interaction with the operator to work, it may be placed along a substantial portion of the length of the catheter. When the embolic coil delivery system 100 is loaded with the embolic coil 110 and the introducer wire 111, the distal end of the introducer wire, proximal to any end fitting 132, defines the distal most preferable position for the locking mechanism. In this position, the locking mechanism may also act as a proximal stop for end fitting 132. As shown in FIG. 1, locking mechanism 114 may, for example, be placed in the proximal third of the catheter. In some contemplated embodiments, locking mechanism 114 may be the proximal most component of catheter 112.

In the embodiment of FIG. 1, catheter 112 and wire 111 have circular cross sections. As such, catheter 112 has an inner diameter that extends for a greater portion of its length and wire 111 has an outer diameter that extends for a greater portion of its length. In some embodiments, it is preferable for wire 111 to have a constant outer diameter for greater portion of its length so that locking mechanism 114 can continue to operate as wire 111 is used to deploy embolic coil 110. In other embodiments, it is not necessary for the locking mechanism 114 to be operable after the delivery wire is moved from its initial configuration. In these embodiments, wire 111 may have a uniform outer diameter over a smaller portion of its length and may include tapers and/or other cross-sectional shapes as desired in certain distal sections of wire 111. Further, where wire 111 has a circular cross section, locking mechanism 114 may be a section of the catheter that has an appropriate reduction in the inner diameter.

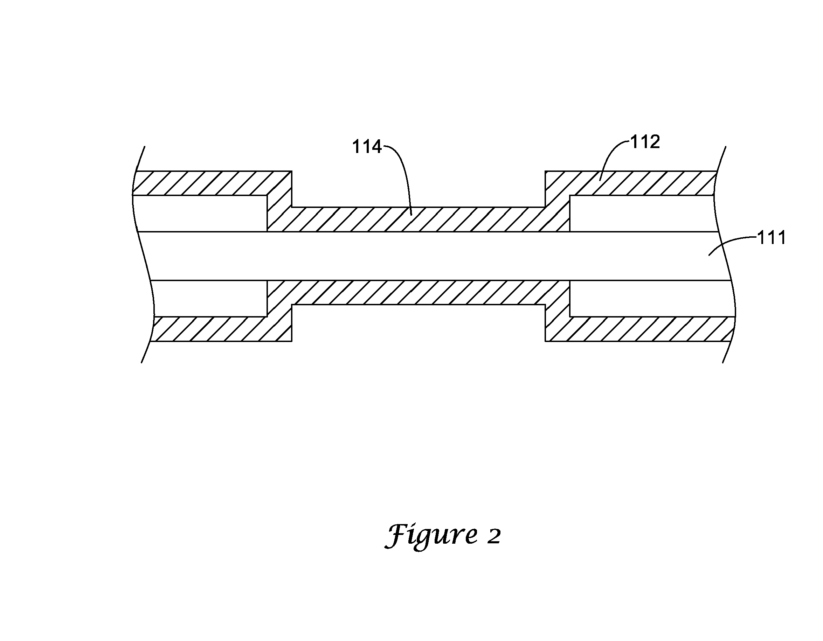

FIG. 2 is a partial cross-sectional view depicting a proximal portion of an embodiment of an embolic coil introducer system including a catheter 112 and introducer wire 111. Locking mechanism 114 is a section of the catheter separated from the greater portion of the catheter by stepwise transitions.

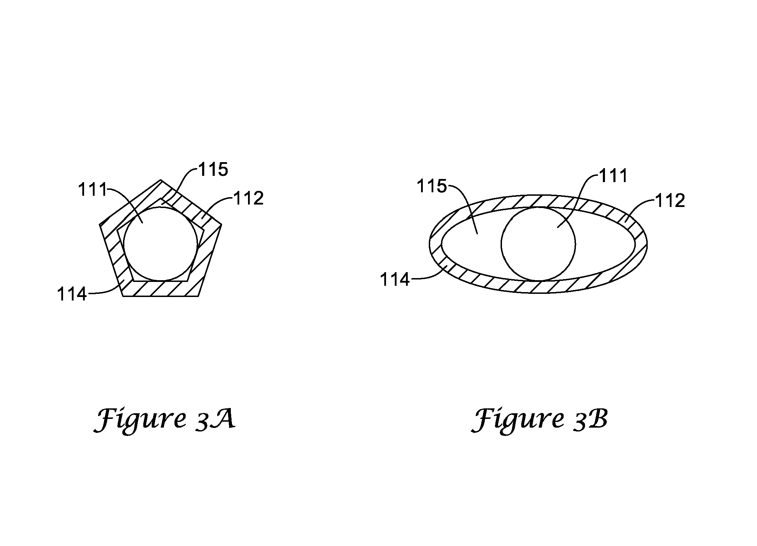

FIGS. 3A and 3B are cross-sectional views of locking mechanisms where the plane of the view extends laterally across the locking mechanism. These embodiments illustrated that the locking mechanism need not have a circular profile or continuous circumferential contact with the wire. In FIG. 3A, an embodiment of locking mechanism 114 is illustrated as defining a polygonal lumen segment 115. With such an embodiment, the locking mechanism has several noncontiguous contact surfaces with the wire that are generally regularly spaced apart in a circumferential manner about the wire. The number, area and location of these contact surfaces determine the level of force applied to the wire. The lumen defined within the locking mechanism may be a wide variety of shapes. For example, the lumen may be star shaped, polygonal, scalloped or any other shape that allows the locking mechanism to function in the manner described herein. For example, the lumen 115 defined by the locking mechanism 114 of FIG. 3B is a generally oval shape having two noncontiguous contact surfaces on opposite sides of the wire 111.

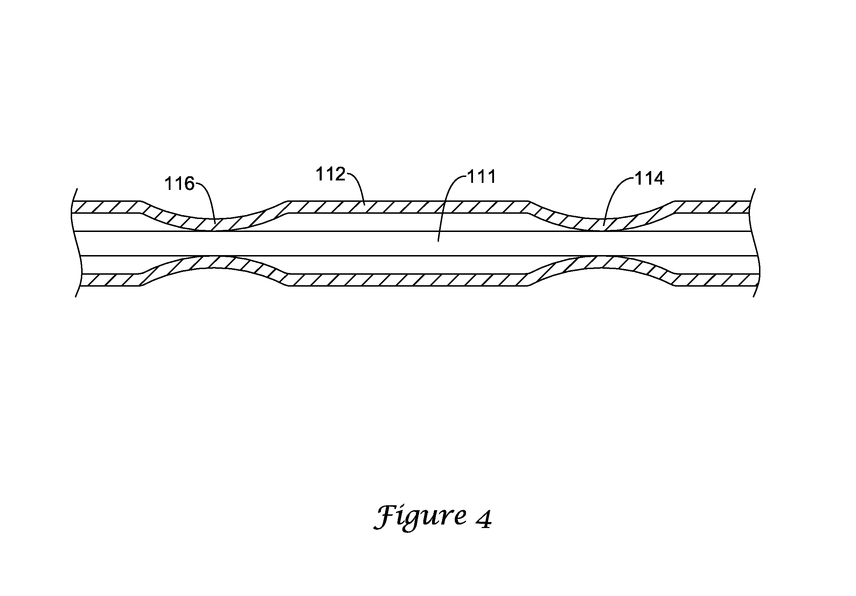

FIG. 4 is a partial cross-sectional schematic view depicting the proximal portion of an embolic coil introducer system where the catheter 112 includes a first locking mechanism 114 and a second locking mechanism 116. The first and second locking mechanisms are preferably spaced apart by a portion of catheter 112 that applies no frictional force to the wire. Embodiments may have additional locking mechanism sections as desired to increase the level of force applied to the wire as needed. Proximal locking mechanism sections may be separated by perforations or the like to allow the operator to remove proximal sections to adjust the level of force to a personally preferred level.

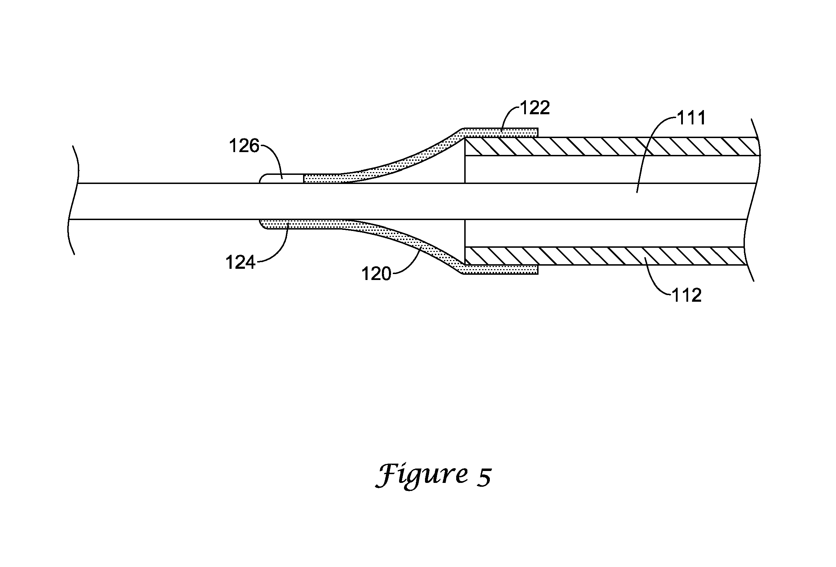

FIG. 5 is a partial cross-sectional schematic view of the proximal portion of an embodiment of an embolic coil introducer system where a removable element 120 blocks wire 111 to catheter 112. Removable element 120 may be a sleeve such as a piece of heat shrink tubing and includes a section 122 gripping catheter 112 and a section 124 gripping wire 111. A slot or perforation 126 may be included at either the distal or proximal end of the removable element to aid in the removal of element 120. Removable element 120 may be included in any of the systems described above that also include a locking mechanism 114.

Embolic coil introducer systems including locking mechanisms as described above are not limited to systems where the introducer wire has a circular cross section. The introducer wire 111 may have, for example, and oblong cross-section. In such a case, the locking mechanism would define a corresponding lumen. The lumen may have been oblong cross-sectional shape as well or it may define several noncontiguous contact surfaces between the locking mechanism and the wire.

Locking mechanisms 114 may be produced by pressing or crimping that sheath to the wire with a pair of heated crimp pliers melting a section of the sheath to produce intimate contact with the wire. The cross-section of the crimped sheath is determined by the design shape of the crimping section of the pliers, with the purpose of producing sufficient adhesion and/or friction force between the sheath and the wire as a way to secure the sheath onto the wire. The adhesion or friction force is determined by factors including the contact area, the shape of the cross-section of the crimped sheath, and the temperature and crimp force applied to the heated pliers. The temperature of the heated pliers is preferably high enough to ensure the crimped section of the sheath is melted. The crimping section of the pliers may have any appropriate shape, including but not limited to a star or an oval shape. In another method of manufacture, the locking mechanism section of the catheter may be produced by extruding a narrower section of the catheter. Other appropriate methods of manufacture may be used as well.

In use, an embolic coil introducer system 110 may be provided having an introducer wire 111 and an embolic coil 110 loaded therein. The system may be advanced as desired within a body lumen, while the locking mechanism 114 retains the relative positions of the catheter and introducer wire. When the desired location is reached, the physician may advance the wire relative to the catheter, without doing anything to the locking mechanism to deploy embolic coil by applying an appropriate degree of force to the wire. When the physician stops applying force to the wire, the force provided by the locking mechanism keeps the wire from moving within the catheter. Typically, the distal end of the introducer wire is advanced out of the distal end of the catheter to release embolic coil. Once the embolic coil is released, the introducer wire may be withdrawn back into the catheter, and the system may be withdrawn from the patient's body. This example of use is intended to show that the locking mechanism of the embodiments described can be used within embolic coil introducer systems without the time or complication of additional steps in the procedure.

Various embodiments of the invention have now been described in detail. Since changes, alterations and additions to the above described embodiments may be made without departing from the nature or scope of the invention, the invention is not to be limited to the embodiments described above, but rather the scope of the invention is defined only by the appended claims. For example, while the invention is described with respect to embolic coil introducer systems, the invention may be used in any catheter system where releasable fixing of an elongate interior member to the catheter is desirable.

## Claims

1. An embolic coil introducer system (110), comprising:
a catheter (112) having a proximal end, a distal end, a lumen extending between the proximal and distal ends and a longitudinal axis;
an embolic coil (110) at least partially disposed in the lumen of the catheter (112); and
a wire (111) for moving the embolic coil (110) at least partially disposed in the lumen of the catheter (112), the wire (111) having a first diameter along a proximal region of the wire (111),
wherein the catheter (112) includes a locking mechanism (114) that provides a friction fit between the catheter (112) and the wire (111) to hold the wire (111) secure relative to the catheter (112) in a first position when no force is applied to the wire (111) and wherein the friction fit may be overcome by the application of a force to the wire (111) to allow the wire (111) to move relative to the catheter (112); **characterised in that** the locking mechanism (114) is formed as a unitary component of the catheter (112) and the locking mechanism (114) is a reduced profile section (122) of the catheter (112).

2. The system of claim 1, wherein the locking mechanism (114) is configured to automatically hold the wire (111) secure relative to the catheter (112) in a second position after force has been applied to the wire (111) to move the wire (111) relative to the catheter (112), after the force has then been removed.

3. The system of one of claim 1 or 2, wherein the locking mechanism (114) is configured such that the force needed to overcome the friction fit is between 0.22 N (0.05 lbs) and 0.58 N (0.13 lbs).

4. The system of one of claim 1-3, wherein the locking mechanism (114) is distal to the proximal end and in the proximal third of the catheter (112).

5. The system of one of claim 1-4, wherein the locking mechanism (114) comprises a lumen for receiving the wire (111) wherein the cross-sectional shape of the lumen is noncircular.

6. The system of one of claim 1-5, wherein the locking mechanism (114) comprises a first section that provides a friction fit with the wire (111) and a second section that provides a friction fit with the wire (111) separated longitudinally from each other by an intermediate section that does not provide a friction fit with the wire (111) or is otherwise fixed to the wire (111).

7. The system of one of claim 1-6, wherein the locking mechanism (114) has only a single configuration.

8. The system of one of claim 1-7, further comprising a removable element (120) disposed at the proximal end of the catheter (112) to fix the relative longitudinal position of the catheter (112) with the wire (111).

9. The system of claim 8, wherein the removable element (120) includes a feature selected from the group consisting of a slit and a perforation (126).

10. The system of one of claim 1, wherein the locking mechanism has essentially the same size and shape in the first position as in a second position where the wire (111) is sliding relative to the catheter (112), and wherein the catheter (112) is locked to the wire (111) in the first position.

11. The system of one of claim 1-10, further comprising an adhesion bond between the locking mechanism (114) and the wire (111) in the first position.

12. The system of one of claim 1-10 further comprising a friction fit between the locking mechanism (114) and the wire (111) in the first position.

13. The system of claim 11, further comprising a friction fit between the locking mechanism (114) and the wire (111) in a third position where the wire (111) is static relative to the catheter (112).

14. The system of one of claim 1-7, wherein contact between the catheter (112) and the wire (111) fixes the wire (111) in place when less than 0.044 N (0.01 lbs of force) are applied to the wire (111).

## Patentansprüche

1. Introducer-System für eine embolische Spirale (110), mit:
einem Katheter (112), der ein proximales Ende, ein distales Ende, ein Lumen, das sich zwischen dem proximalen und dem distalen Ende erstreckt, und eine Längsachse aufweist;
einer embolischen Spirale (110), die mindestens teilweise im Lumen des Katheters (112) angeordnet ist; und
einem Draht (111) zum Bewegen der embolischen Spirale (110), die mindestens teilweise im Lumen des Katheters (112) angeordnet ist, wobei der Draht (111) längs eines proximalen Bereichs des Drahtes (111) einen ersten Durchmesser aufweist,
wobei der Katheter (112) einen Arretierungsmechanismus (114) aufweist, der einen Reibsitz zwischen dem Katheter (112) und dem Draht (111) bereitstellt, um den Draht (111) relativ zum Katheter (112) sicher in einer ersten Position zu halten, wenn keine Kraft auf den Draht (111) ausgeübt wird, und wobei der Reibsitz durch die Anwendung einer Kraft auf den Draht (111) überwunden werden kann, um es dem Draht (111) zu ermöglichen, sich relativ zum Katheter (112) zu bewegen;
**dadurch gekennzeichnet, dass**
der Arretierungsmechanismus (114) als eine einheitliche Komponente des Katheters (112) ausgebildet ist und der Arretierungsmechanismus (114) ein reduzierter Profilabschnitt (122) des Katheters (112) ist.

2. System nach Anspruch 1, wobei der Arretierungsmechanismus (114) konfiguriert ist, den Draht (111) automatisch relativ zum Katheter (112) sicher in einer zweiten Position zu halten, nachdem eine Kraft auf den Draht (111) ausgeübt worden ist, um den Draht (111) relativ zum Katheter (112) zu bewegen, nachdem die Kraft gelöst worden ist.

3. System nach Anspruch 1 oder 2, wobei der Arretierungsmechanismus (114) so konfiguriert ist, dass die zum Überwinden des Reibsitzes benötigte Kraft zwischen 0,22 N (0,05 lbs) und 0,58 N (0,13 lbs) liegt.

4. System nach einem der Ansprüche 1 bis 3, wobei der Arretierungsmechanismus (114) sich distal vom proximalen Ende und im proximalen Drittel des Katheters (112) befindet.

5. System nach einem der Ansprüche 1 bis 4, wobei der Arretierungsmechanismus (114) ein Lumen zum Aufnehmen des Drahtes (111) aufweist, wobei die Querschnittsform des Lumens nicht kreisförmig ist.

6. System nach einem der Ansprüche 1 bis 5, wobei der Arretierungsmechanismus (114) einen ersten Abschnitt, der einen Reibsitz mit dem Draht (111) bereitstellt, und einen zweiten Abschnitt aufweist, der einen Reibsitz mit dem Draht (111) bereitstellt, die durch einen Zwischenabschnitt longitudinal voneinander getrennt sind, der keinen Reibsitz mit dem Draht (111) bereitstellt oder auf andere Weise am Draht (111) befestigt ist.

7. System nach einem der Ansprüche 1 bis 6, wobei der Arretierungsmechanismus (114) nur eine einzige Konfiguration aufweist.

8. System nach einem der Ansprüche 1 bis 7, das ferner ein entfernbares Element (120) aufweist, das am proximalen Ende des Katheters (112) angeordnet ist, um die relative longitudinale Position des Katheters (112) mit dem Draht (111) zu fixieren.

9. System nach Anspruch 8, wobei das entfernbare Element (120) ein Merkmal aufweist, das aus der Gruppe ausgewählt ist, die aus einem Schlitz und einer Perforation (126) besteht.

10. System nach Anspruch 1, wobei der Arretierungsmechanismus in der ersten Position im Wesentlichen dieselbe Größe und Form wie in einer zweiten Position aufweist, wo der Draht (111) relativ zum Katheter (112) gleitet, und wobei der Katheter (112) in der ersten Position zum Draht (111) arretiert ist.

11. System nach einem der Ansprüche 1 bis 10, das ferner in der ersten Position eine Adhäsionsbindung zwischen dem Arretierungsmechanismus (114) und dem Draht (111) aufweist.

12. System nach einem der Ansprüche 1 bis 10, das ferner in der ersten Position einen Reibsitz zwischen dem Arretierungsmechanismus (114) und dem Draht (111) aufweist.

13. System nach Anspruch 11, das ferner in einer dritten Position, in der der Draht (111) relativ zum Katheter (112) statisch ist, einen Reibsitz zwischen dem Arretierungsmechanismus (114) und dem Draht (111) aufweist.

14. System nach einem der Ansprüche 1 bis 7, wobei ein Kontakt zwischen dem Katheter (112) und dem Draht (111) den Draht (111) an Ort und Stelle fixiert, wenn eine Kraft von weniger als 0,044 N (0,01 lbs) auf den Draht (111) ausgeübt wird.

## Revendications

1. Système d'introduction d'une bobine embolique (110), comprenant :
un cathéter (112) présentant une extrémité proximale, une extrémité distale, une lumière s'étendant entre l'extrémité proximale et l'extrémité distale, et un axe longitudinal ;
une bobine embolique (110) au moins partiellement disposée dans la lumière du cathéter (112) ; et
un fil (111) pour déplacer la bobine embolique (110) au moins partiellement disposée dans la lumière du cathéter (112), le fil (111) présentant un premier diamètre le long d'une zone proximale du fil (111),
où le cathéter (112) comprend un mécanisme de verrouillage (114) réalisant un ajustement par friction entre le cathéter (112) et le fil (111) pour fixer le fil (111) dans une première position par rapport au cathéter (112) quand aucune force n'est appliquée sur
le fil (111), et où l'ajustement par friction peut être desserré par application d'une force sur le fil (111) pour permettre le déplacement du fil (111) par rapport au cathéter (112) ;
**caractérisé en ce que**
le mécanisme de verrouillage (114) est réalisé comme un composant unitaire du cathéter (112), et **en ce que** le mécanisme de verrouillage (114) est une section à profil rétréci (122) du cathéter (112).

2. Système selon la revendication 1, où le mécanisme de verrouillage (114) est prévu pour fixer automatiquement le fil (111) dans une deuxième position par rapport au cathéter (112) par application d'une force sur le fil (111) pour déplacer le fil (111) par rapport au cathéter (112), après relâchement de la force.

3. Système selon la revendication 1 ou la revendication 2, où le mécanisme de verrouillage (114) est prévu pour que la force exigée pour desserrer l'ajustement par friction soit comprise entre 0,22 N (0,05 lbs) et 0,58 N (0,13 lbs).

4. Système selon l'une des revendications 1 à 3, où le mécanisme de verrouillage (114) est distal par rapport à l'extrémité proximale et disposé dans le tiers proximal du cathéter (112).

5. Système selon l'une des revendications 1 à 4, où le mécanisme de verrouillage (114) comprend une lumière pour recevoir le fil (111), la lumière ayant une section transversale de forme non circulaire.

6. Système selon l'une des revendications 1 à 5, où le mécanisme de verrouillage (114) comprend un premier segment assurant un ajustement par friction avec le fil (111) et un deuxième segment assurant un ajustement par friction avec le fil (111), séparés longitudinalement l'un de l'autre par un segment intermédiaire n'assurant pas d'ajustement par friction avec le fil (111) ou fixé d'une autre manière au fil (111).

7. Système selon l'une des revendications 1 à 6, où le mécanisme de verrouillage (114) ne présente qu'une configuration simple.

8. Système selon l'une des revendications 1 à 7, comprenant en outre un élément amovible (120) disposé à l'extrémité proximale du cathéter (112) pour fixer la position longitudinale relative du cathéter (112) et du fil (111).

9. Système selon la revendication 8, où l'élément amovible (120) présente une caractéristique sélectionnée dans le groupe composé d'une fente et d'une perforation (126).

10. Système selon la revendication 1, où le mécanisme de verrouillage a sensiblement la même forme et la même dimension dans la première position que dans une deuxième position où le fil (111) est coulissant par rapport au cathéter (112), et où le cathéter (112) est verrouillé sur le fil (111) dans la première position.

11. Système selon l'une des revendications 1 à 10, comprenant en outre une liaison adhésive entre le mécanisme de verrouillage (114) et le fil (111) dans la première position.

12. Système selon l'une des revendications 1 à 10, comprenant en outre un ajustement par friction entre le mécanisme de verrouillage (114) et le fil (111) dans la première position.

13. Système selon la revendication 11, comprenant en outre un ajustement par friction entre le mécanisme de verrouillage (114) et le fil (111) dans une troisième position où le fil (111) est statique par rapport au cathéter (112).

14. Système selon l'une des revendications 1 à 7, où un contact entre le cathéter (112) et le fil (111) maintient le fil (111) en place si une force inférieure à 0,044 N (0,01 lbs) est appliquée sur le fil (111).
